# EUROPEAN PATENT SPECIFICATION

(11) **EP 4 338 992 B1**
(45) Date of publication and mention of the grant of the patent: **29.10.2025**
(21) Application number: 22196307.7
(22) Date of filing: 19.09.2022
(51) Int. Cl.: B60H 1/24, B60H 3/02, B60H 3/06, B01J 20/28, B60H 1/32, F25B 15/00, B01D 53/04, B01D 46/121, B01D 46/00

(54) **SORPTION FILTER ELEMENT**
SORPTIONSFILTERELEMENT
ÉLÉMENT FILTRANT À SORPTION

(43) Date of publication of application: 20.03.2024
(73) Proprietor: MANN+HUMMEL GmbH, 71636 Ludwigsburg (DE)
(72) Inventor: MBADINGA MOUANDA, Gelase, 74343 Sachsenheim (DE); EVLEKLI, Coskun, 71332 Waiblingen (DE); PROCHAZKA, Josef, 59401 Velke Mezirici (CZ)
(74) Representative: Mann + Hummel Intellectual Property

(56) References cited:
- WO-A1-99/02242
- US-A1- 2022 016 567
- US-B1- 10 870 076

## Description

### Technical field

The present invention relates to a sorption filter element for a sorption filter device.

### Prior art

In the case of vehicles that are at least partially electrically powered, it is advisable to save as much energy as possible in order to achieve the longest possible range. For reasons of comfort, the passenger compartment of such a vehicle can usually be air conditioned with the aid of an air conditioning system. With regard to the desired energy savings, it is advantageous if the air conditioning system for air conditioning the passenger compartment draws in as little fresh air as possible from the vehicle's surroundings and heats or cools it, but instead recirculates and air conditions the air in the passenger compartment.

In the aforementioned recirculation mode for air conditioning the passenger compartment, however, water contained in the exhaled air of occupants or passengers can accumulate in the passenger compartment, which can lead to fogging of windows, for example a windshield or side windows, of the motor vehicle. The reason for this is that the dehumidifying effect of an air conditioning system, which results from a dew point undershoot in the evaporator heat exchanger, cannot be used in recirculation mode. This must be avoided or at least reduced with regard to safety aspects.

Furthermore, carbon dioxide contained in the exhaled air can also accumulate in the passenger compartment. This can lead to concentration problems or even health problems for the occupants. This, too, must be prevented or at least reduced with regard to both safety and health aspects, since in the worst-case scenario, an excessively high concentration of carbon dioxide in the passenger compartment air can reduce the driver's ability to concentrate to such an extent that accidents are imminent.

US 10 870 076 B1 describes an air filtration system, an air filtration device, and an air filtration module for removing ultra-fine particles (UFPs), pathogens (e.g., viruses, bacteria, etc.), volatile organic compounds (VOCs), oxides or odors. The air filtration system includes a docking base for receiving the removable, portable air filtration device in fluid communication with the docking base. In certain embodiments, the air filtration system further includes the removable, portable air filtration device. The portable air filtration device may optionally include the air filtration module including at least two primary filter media, wherein the at least two primary filter media are secured to an internal chassis in a spaced apart orientation in a parallel air flow configuration.

US 2022/016567 A1 describes a device for reducing a carbon dioxide and water content in an enclosed air volume and having first and second sorption units for sorbing carbon dioxide and water. The first and second sorption units can be transferred from a sorption mode into a desorption mode and vice versa. In the sorption mode, the first and second sorption units sorb carbon dioxide and water from raw air of the enclosed air volume. In the desorption mode, the first and second desorption units desorb carbon dioxide and water to supplied regeneration air. An air distribution device can switch the first and second sorption units, based on the carbon dioxide and water content, alternately from sorption mode into desorption mode such that. In at least one operating state of the device, one of the first and second sorption units is in sorption mode while the other is in desorption mode.

### Disclosure of the invention

It is therefore an object of the invention to provide an improved sorption filter element for a mechanism for reducing the carbon dioxide and water content in a passenger compartment of a motor vehicle.

Accordingly, a sorption filter element for a sorption filter device is provided. The sorption filter element comprises at least two sorption bodies with at least one sorbent, wherein the sorption bodies are arranged in a V-shape, wherein the sorption bodies enclose an intermediate space that is open at a head side thereof, wherein the sorption filter element comprises an outer sealing element that runs around an outer circumference of the head side, wherein the sorption filter element comprises a circumferential inner sealing surface that is arranged at the head side, wherein the inner sealing surface runs around a circumferene delimiting the intermediate space at the head side. The inner sealing surface is axially set back relative to the outer sealing element.

The inner sealing surface is placed within the intermediate space, in particular in a region of the intermediate space that is close to the head side.

The sorption filter element is particularly suitable for adsorbing and/or absorbing carbon dioxide and water. However, also substances like nitrogen oxides (NOX) and/or volatile organic compounds (VOCs) can be adsorbed and/or absorbed by the sorption filter element. Examples of volatile organic compounds are higher hydrocarbons. The sorption filter element may also be suitable for adsorbing and/or absorbing sulfur dioxide (SO2).

In the present context, "sorption" refers to processes that lead to an enrichment of a substance, for example carbon dioxide or water, within a phase or on an interface between two phases. Accumulation within a phase is referred to as absorption, while accumulation at the interface is referred to as adsorption. In the present context, "desorption" refers to processes in which atoms or molecules, in particular carbon dioxide or water, leave the surface of a solid. Desorption thus generally represents the reverse process of sorption. By applying heat to the sorbent, the adsorbed and/or absorbed substances can be released from the sorbent.

In a sorption mode, the sorption filter element adsorbs and/or absorbs different substances like carbon dioxide and/or water. In a desorption mode, the enriched substances can be released from the sorption filter element. Thus, the sorption filter element can be regenerated. In the desorption mode, heat is applied to the sorption filter element, in particular to the sorbent. The sorption filter element may be a pure adsorption filter element or may be designated as such. Preferably, the sorption filter element can be easily and quickly replaced. The sorption mode may also be, or be designated as, a pure adsorption mode. The desorption mode may also be referred to as regeneration mode.

The sorption filter element preferably comprises several sorbents. In particular, a sorbent suitable to sorb, especially to adsorb, carbon dioxide may be provided. This sorbent may be referred to as carbon dioxide sorbent or CO2 sorbent. Another sorbent may be provided that is suitable to sorb, especially adsorb, water. This sorbent may be referred to as water sorbent or H2O sorbent. The sorbents may be in granular or fibrous form, in particular in bulk form. In particular, the sorbents are fixed by means of a carrier material. The sorbents may also be pure adsorbents or be designated as such. Raw air may be guided through the sorbents in a parallel or serial way.

In the desorption mode, regeneration air can be taken from an enclosed air volume or from an environment. Initially, the regeneration air is unloaded. In the desorption mode, the unloaded regeneration air is loaded with carbon dioxide and water and supplied to the environment as loaded regeneration air. By the fact that the regeneration air is "unloaded" with carbon dioxide and water, it is to be understood in particular that the regeneration air can absorb stored carbon dioxide and water in the sorption filter element being in desorption mode. However, this does not preclude the unloaded regeneration air from also being able to absorb a certain amount of carbon dioxide and water. However, the unloaded regeneration air is not saturated with carbon dioxide and water.

The sorption bodies being arranged in a V-shape in this context means that the sorption bodies are arranged inclined to each other. The intermediate space is provided between the sorption bodies resulting in a wedge-shaped geometry of the intermediate space. The "head side" of the intermediate space is a side of the intermediate space that faces away from the sorption bodies. The outer sealing element is made of a flexible material. The outer sealing element runs around the open head side. Preferably, the inner sealing surface is arranged within the outer sealing element.

Due to the outer sealing element and the inner sealing surface, a unique sealing interface of the sorption filter element can be created. This prevents incorrect installation of the sorption filter element or replacement with a sorption filter element that does not fit the sorption filter device.

In embodiments, the sorption filter element comprises two spaced face walls, wherein in particular the sorption bodies are connected to each other by means of face walls. Preferably, there are provided two face walls that sandwich the sorption bodies.

In embodiments, the inner sealing surface is formed at least partially by a collar section of the face walls being drawn into the intermediate space on an inside thereof.

In embodiments, the sorption filter element comprises at least two grid-like side walls, wherein each side wall neighbors at least one of the sorption bodies and/or at least partially encloses the sorbent. Thus, the side walls are air permeable.

In embodiments, the side walls are covered with an air permeable material that faces the sorbent, wherein the material holds back the sorbent. The material can be a membrane or the like.

In embodiments, the face walls and the side walls are formed integrally. The face walls and the side walls are part of a body of the sorption filter element. Preferably, the body is an injection molded part.

In embodiments the sorption filter element comprises a base body comprising at least the face walls and the side walls, wherein the sorption bodies are replaceably held at the base body, in particular between the face walls and neighboring at least one of the side walls. The sorption filter element can comprise a connection interface between the sorption bodies and the base body, wherein the connection interface can comprise a connection means that detachably connects the sorption bodies to the base body. The connection means can comprise at least one hinge means and/or at least one snap-fit means.

In embodiments, at least one of the face walls comprises a guidance track, wherein the guidance track is configured to engage with a housing-sided guidance track for axially guiding the sorption filter element.

In embodiments, the outer sealing element and/or the inner sealing surface is rectangular. Alternatively, the outer sealing element and/or the inner sealing surface can have any arbitrary shape.

In embodiments, the sorbent is bulk material. The sorbent can be sphere-shaped

In embodiments, the sorption filter element comprises a first sorbent and a second sorbent.

In embodiments, the first sorbent is mixed with the second sorbent.

In embodiments, the first sorbent and the second sorbent are provided in layers being separated from each other by means of an air permeable separating material. The separating material can be a membrane or the like.

Furthermore, a sorption filter device is provided. The sorption filter device comprises at least one sorption filter element, and a housing, wherein the housing comprises a first housing part for receiving the sorption filter element and a second housing part, wherein the second housing part is detachably connectable to the first housing part, wherein the second housing part closes the housing in a closed state thereof, wherein the sorption filter element is received in the first housing part in such a way that the head side faces the second housing part, wherein the housing comprises at least one first air inlet/outlet and at least one second air inlet/outlet, wherein the sorption filter element is arranged between the first air inlet/outlet and the second air inlet/outlet in a fluidic way, wherein at least one of the first air inlet/outlet and the second air inlet/outlet is provided at the second housing part, wherein the sorption filter device comprises an air guidance element that is arranged between the sorption filter element and the second housing part, and wherein the air guidance element protrudes into the intermediate space at the head side in a mounted state of the second housing part.

Furthermore, use of a sorption filter element in a sorption filter device is provided.

Furthermore, use of a sorption filter device in a system for reducing the CO2-content within a passenger compartment is provided.

### Short description of the drawings

The figures show:
- Fig. 1: a schematic view of one embodiment of a motor vehicle;
- Fig. 2: a schematic view of one embodiment of a mechanism for the motor vehicle according to Fig. 1;
- Fig. 3: a schematic perspective view of one embodiment of a sorption filter device for the mechanism according to Fig. 2;
- Fig. 4: a schematic exploded perspective view of the sorption filter device according to Fig. 3;
- Fig. 5: a schematic top view of the sorption filter device according to Fig. 3;
- Fig. 6: a schematic cross-sectional view of the sorption filter device according to the intersection line VI-VI of Fig. 5;
- Fig. 7: a further schematic cross-sectional view of the sorption filter device according to the intersection line VII-VII of Fig. 6;
- Fig. 8: a further schematic cross-sectional view of the sorption filter device according to Fig. 3;
- Fig. 9: a schematic perspective view of one embodiment of a second housing part for the sorption filter device according to Fig. 3;
- Fig. 10: a schematic exploded perspective view of the second housing part according to Fig. 9;
- Fig. 11: a schematic perspective view of one embodiment of an air heating and guidance mechanism for the sorption filter device according to Fig. 3;
- Fig. 12: a schematic perspective view of one embodiment of a sorption filter element for the sorption filter device according to Fig. 3; and
- Fig. 13: a schematic exploded perspective view of the sorption filter element according to Fig. 12.

In the figures, identical or functionally identical elements have been given the same reference signs unless otherwise indicated.

### Embodiment(s) of the invention

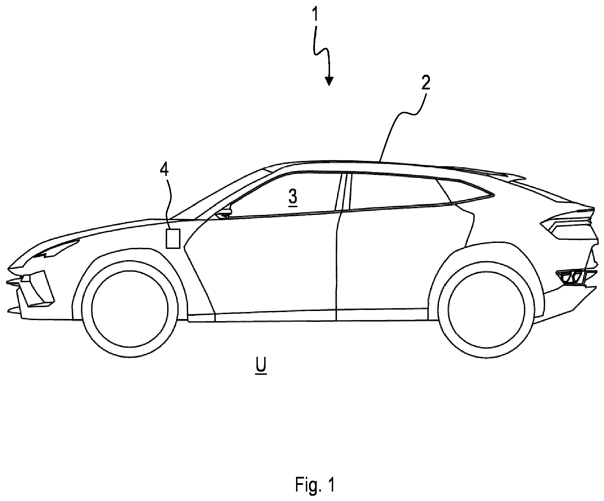

Fig. 1 shows a schematic view of an embodiment of a motor vehicle 1. The motor vehicle 1 is preferably an electric vehicle or a hybrid vehicle. However, the motor vehicle 1 may also be driven by means of an internal combustion engine. The motor vehicle 1 comprises a body 2 which encloses an enclosed air volume 3. "Enclosed" in this context means that the body 2 defines boundaries or a geometric extension of the enclosed air volume 3. However, this does not necessarily mean that the enclosed air volume 3 cannot be in air exchange with an environment U of the motor vehicle 1.

The enclosed air volume 3 is an interior or passenger compartment of the motor vehicle 1. However, the enclosed air volume 3 may also be associated with a watercraft, a construction machine or a construction vehicle, a rail vehicle, an agricultural machine or an agricultural vehicle, or an aircraft. However, the enclosed air volume 3 may also be part of a building or a stationary machine.

In the following, however, it is assumed that the enclosed air volume 3 is the passenger compartment of the motor vehicle 1. Therefore, the enclosed air volume 3 is hereinafter referred to as the passenger compartment. The passenger compartment 3 can be air conditioned by means of a heating, ventilation, and air conditioning (HVAC) system 4. To extend a range of such an electrically driven motor vehicle 1, it is desirable to save as much energy as possible. With reference to the HVAC system 4, this means that it should draw in as little fresh air as possible from the environment U of the motor vehicle 1 to air condition the passenger compartment 3.

However, when recirculated air taken in the passenger compartment 3 is used to air condition the passenger compartment 3, water (H2O) contained in the exhaled air of occupants or passengers may accumulate in the passenger compartment 3, which may cause windows, for example a windshield or side windows, of the motor vehicle 1 to fog. This must be avoided or at least reduced with regard to safety aspects. Furthermore, carbon dioxide (CO2) contained in the exhaled air can also accumulate in the passenger compartment 3. This can lead to concentration problems or even health impairments for the occupants. This, too, must be prevented or at least reduced with regard to both safety and health aspects.

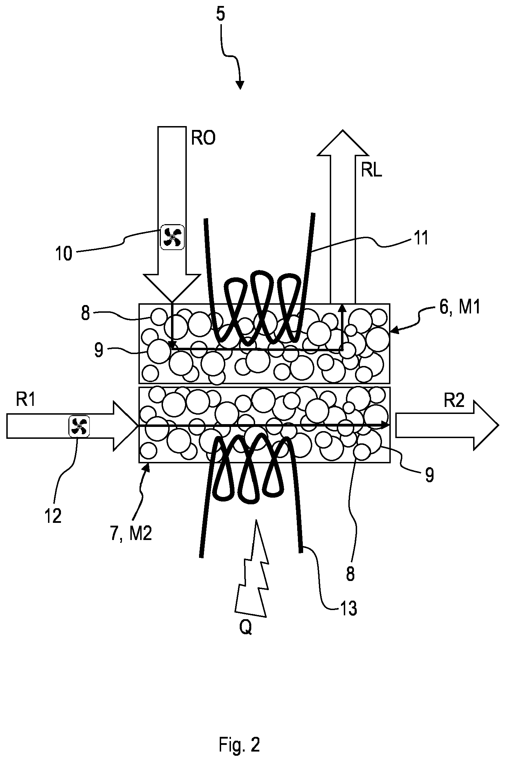

Fig. 2 shows a schematic view of an embodiment of a mechanism 5 for the combined reduction of CO2 and H2O, in particular water vapor, within the passenger compartment 3. With the aid of the mechanism 5, the previously mentioned disadvantages can be prevented or their effect at least reduced. In addition, the mechanism 5 can also be used to prevent impurities from the environment U from entering the passenger compartment 3, since the preparation of the circulating air means that the intake of ambient air can be largely dispensed with.

The mechanism 5 comprises a first sorption filter device 6 and a second sorption filter device 7. "Sorption" is a collective term for processes that lead to an accumulation of a substance within a phase or at an interface between two phases. The enrichment within a phase is more precisely called absorption, and that at the interface adsorption. This means that the sorption filter devices 6, 7 are suitable for adsorbing and/or absorbing substances such as CO2 and H2O, but also nitrogen oxides (NOX) and/or volatile organic compounds (VOCs). Examples of volatile organic compounds are higher hydrocarbons. The sorption filter devices 6, 7 may also be suitable for adsorbing and/or absorbing sulfur dioxide (SO2). Preferably, however, the sorption filter devices 6, 7 are pure adsorbing units or may be designated as such.

The sorption filter devices 6, 7 are preferably interchangeable and, as will be explained further below, can be operated alternately in a sorption mode M1 and in a desorption mode M2. The sorption filter devices 6, 7 are cartridge-shaped and may be referred to as cartridges or sorption filter elements. The sorption mode M1 may also be referred to as the adsorption mode. The desorption mode M2 may also be referred to as regeneration mode. That is, the first sorption filter device 6 is in the sorption mode M1 when the second sorption filter device 7 is in the desorption mode M2 and vice versa. Thus, both sorption filter devices 6, 7 are never in the same mode M1, M2 at the same time. Preferably, the sorption filter devices 6, 7 each have a cartridge shape so that they can be easily exchanged.

Each sorption filter device 6, 7 comprises a first sorbent 8 and a second sorbent 9. Preferably, the sorbents 8, 9 are adsorbents or can be designated as such. For example, the first sorbent 8 is suitable for adsorbing CO2. Accordingly, the second sorbent 9 may be suitable to adsorb H2O. The two sorbents 8, 9 may thus remove H2O and CO2 from the passenger compartment 3. The first sorbent 8 is illustrated with small circles. The second sorbent 9 is illustrated with large circles. Other sorbents may also be provided, suitable for sorbing NOX or VOCs, for example. Thus, any number of different sorbents 8, 9 can be provided for processing the circulating air in the passenger compartment 3.

For example, in addition to the sorbents 8, 9, at least one further sorbent or also several further sorbents (not shown) are provided which are suitable for removing fine particles, NOx and/or VOCs from raw air RO. The further sorbents may be introduced between two carrier layers, in particular carrier layers made of a nonwoven fabric. Alternatively, the sorbents 8, 9 and the further sorbents may be mixed together. The further sorbents may comprise activated carbon which, in particular in the form of a bulk, is preferably introduced between the two carrier layers. The further sorbent or sorbents may be mixed with the first sorbent 8 and/or with the second sorbent 9 to build up one or more mixed bulk layers.

Furthermore, the sorption filter devices 6, 7 can also be suitable for removing allergens, bacteria and/or viruses from the raw air RO in addition to CO2 and H2O. This can be done by a functional coating of carrier materials, for example nonwovens, or by a functional coating of the sorbents 8, 9. Furthermore, the sorption filter devices 6, 7 can have at least one fragrance component from a comfort aspect.

The sorbents 8, 9 can each be in the form of spherical granules. Preferably, the sorbents 8, 9 are fixed to a carrier material or are fixed with the aid of a carrier material. The sorption filter devices 6, 7 can each have a cylindrical, in particular a hollow-cylindrical, a pie-shaped or a rectangular geometry. By a "pie-shaped" geometry is meant in particular a flat circular-cylindrical geometry.

In Fig. 2, the first sorption filter device 6 is in the previously mentioned sorption mode M1. The second sorption filter device 7 is in the desorption mode M2. In the sorption mode M1, raw air RO loaded with CO2 and H2O from the passenger compartment 3 is fed to the first sorption filter device 6. A first blower 10 may be provided for this purpose. The raw air RO is passed through the first sorption filter device 6 at least in sections, with the sorbents 8, 9 purifying the raw air RO of CO2 and H2O. The purified raw air RO is fed back to the passenger compartment 3 as clean air RL.

An optional first heating element 11 is assigned to the first sorption filter device 6, with the aid of which heat Q can be supplied to the sorbents 8, 9. In the sorption mode M1, the first heating element 11 is inactive, so that it does not supply heat Q to the first sorption filter device 6. The first heating element 11 can be a heating wire passed through the first sorption filter device 6, which is energized to introduce heat Q and thus heats the sorbents 8, 9. However, heat Q may also be introduced by any other means. For example, heat Q may be waste heat from an electric motor used to drive the motor vehicle 1. Heat Q may also be waste heat from a conventional internal combustion engine, a battery cooling system, or a fuel cell cooling circuit. The first heating element 11 may also be a heat exchanger or comprise a heat exchanger.

In the desorption mode M2, unloaded regeneration air R1 is supplied to the second sorption filter device 7 being loaded with CO2 and H2O. A second blower 12 can be used for this purpose. By the fact that the unloaded regeneration air R1 is "unloaded" with CO2 and H2O, it is to be understood that the unloaded regeneration air R1 can absorb CO2 and H2O stored in the second sorption filter device 7. That is, the unloaded regeneration air R1 may also have some content of CO2 and H2O. However, the unloaded regeneration air R1 is not saturated with CO2 and H2O. The unloaded regeneration air R1 can be taken from the passenger compartment 3 or the environment U, for example.

Furthermore, an optional second heating element 13 is provided, by means of which the second sorption filter device 7 is heated in the desorption mode M2 and thus heat Q is introduced into the second sorption filter device 7. The first heating element 11 and the second heating element 13 are preferably of identical construction and are operated alternately. As previously mentioned, heat Q may also be supplied, for example, in the form of waste heat from an electric motor. However, as also mentioned previously, heat Q may also be waste heat from a conventional internal combustion engine, a battery cooling system or a fuel cell cooling circuit. The second heating element 13 may also be a heat exchanger or comprise a heat exchanger.

When the sorbents 8, 9 are heated in the desorption mode M2 of the second sorption filter device 7, they release CO2 and H2O to the unloaded regeneration air R1. In other words, CO2 and H2O are desorbed. A temperature above 55 °C is preferably required for desorption. The unloaded regeneration air R1 is passed through the second sorption filter device 7, absorbs CO2 and H2O there and is discharged from the second sorption filter device 7 as loaded regeneration air R2. In particular, the loaded regeneration air R2 is supplied to the environment U.

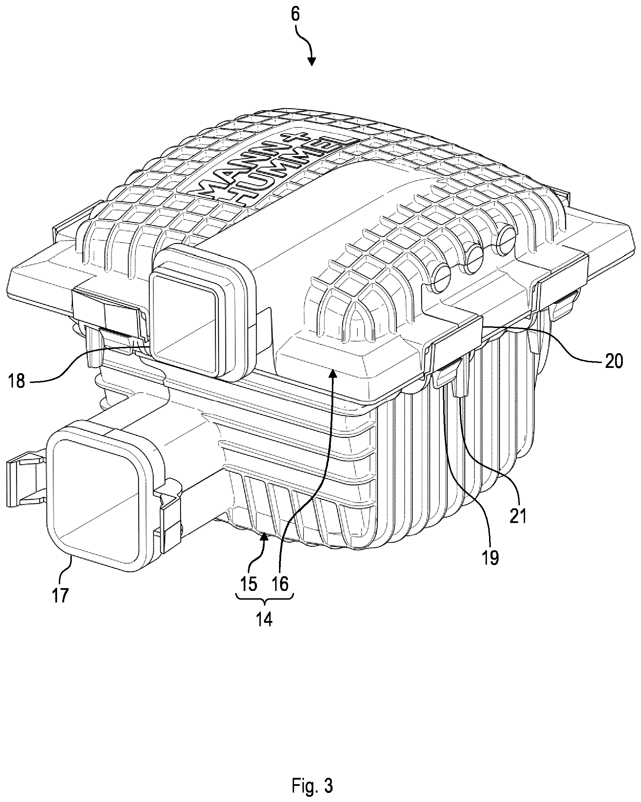

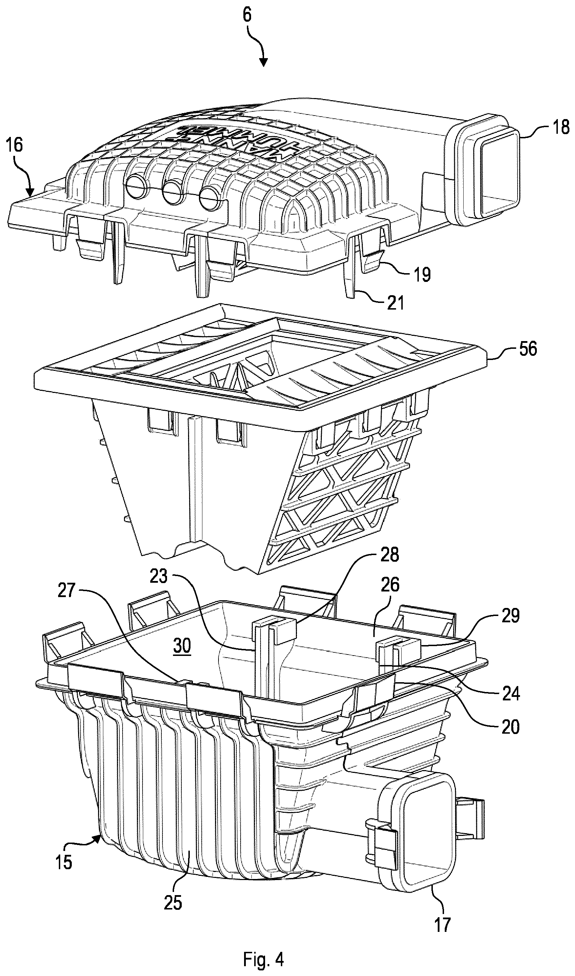

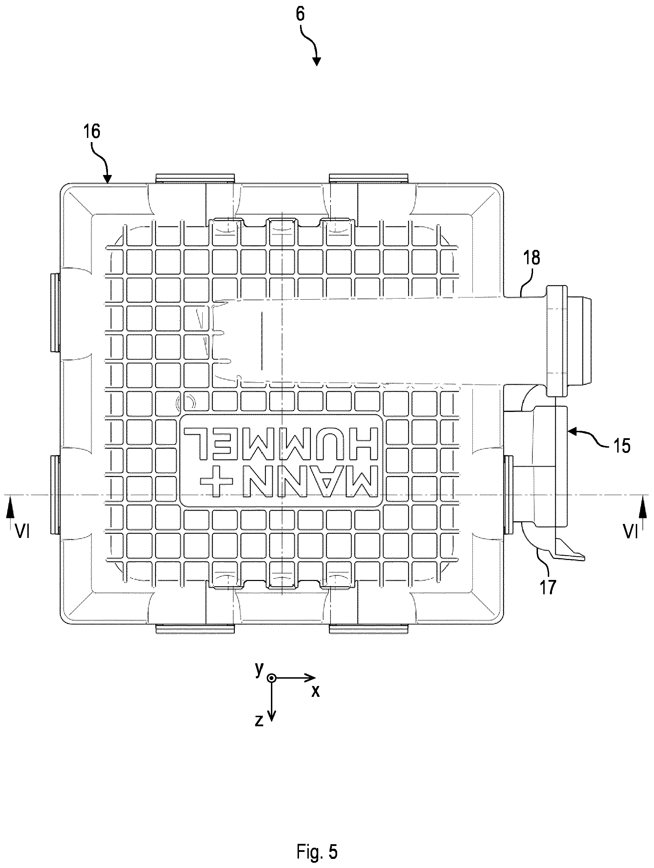

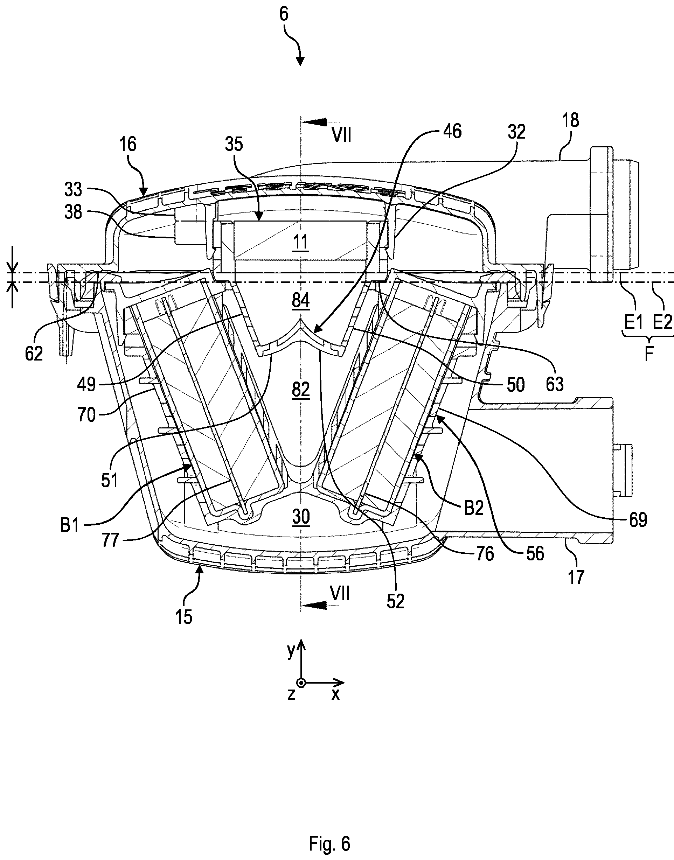

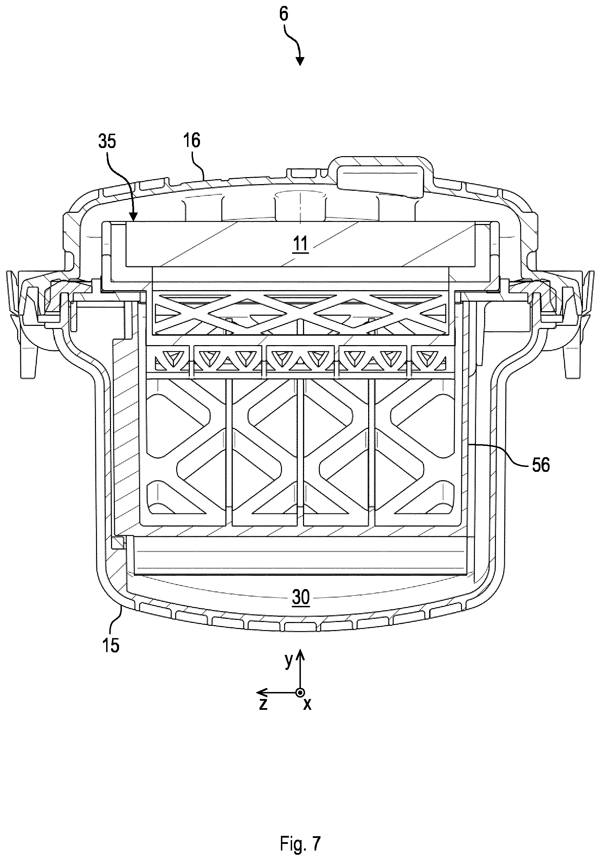

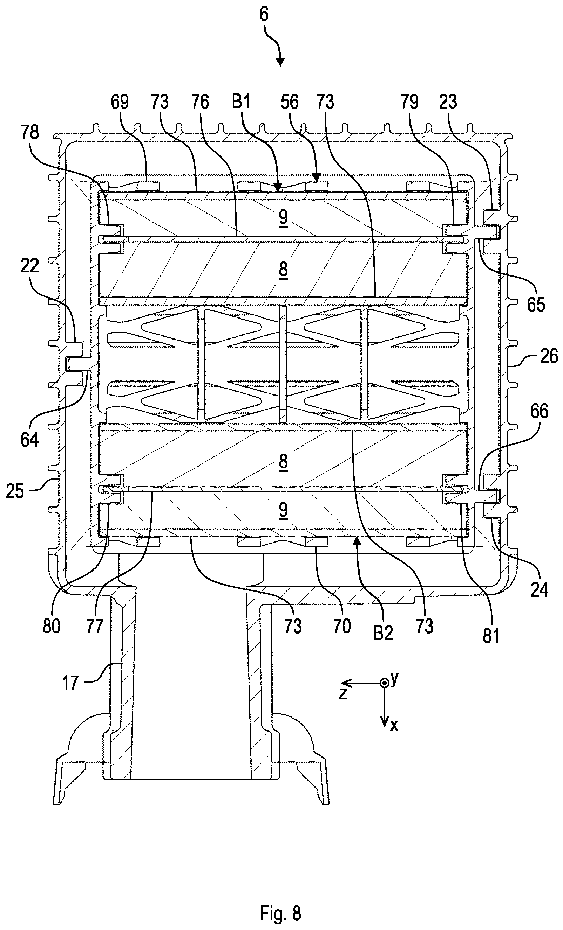

Fig. 3 shows a perspective view of one embodiment of the first sorption filter device 6. Fig. 4 shows a perspective exploded view of the first sorption filter device 6. Fig. 5 shows a top view of the first sorption filter device 6. Fig. 6 shows a cross-sectional view of the first sorption filter device 6 along the intersection line VI-VI of Fig. 5. Fig. 7 shows a further cross-sectional view of the first sorption filter device 6 along the intersection line VII-VII of Fig. 6. Fig. 8 shows a further cross sectional view of the first sorption filter device 6. In the following, Figs. 3 to 8 will be referred to at the same time.

The sorption filter devices 6, 7 are designed identical. In the following, only the first sorption filter device 6 will be referred to. However, all explanations concerning the first sorption filter device 6 can be applied to the second sorption filter device 7 and vice versa.

The first sorption filter device 6 comprises a coordinate system with a width direction or x-direction x, a height direction or y-direction y and a depth direction or z-direction z. The directions x, y, z are arranged perpendicular to each other.

The first sorption filter device 6 comprises a housing 14 with a first housing part 15 and a second housing part 16. The second housing part 16 is attached to the first housing part 15. The second housing part 16 can be removed from the first housing part 15. The first housing part 15 and the second housing part 16 can be plastic parts, in particular injection molded plastic parts. The housing 14 has a first air inlet/outlet 17 and a second air inlet/outlet 18. The first air inlet/outlet 17 is provided at the first housing part 15. The second air inlet/outlet 18 is provided at the second housing part 16. The clean air RL, the raw air RO, and the regeneration air R1, R2 can be guided through the air inlets/ outlets 17, 18 as will be explained later.

The second housing part 16 comprises a plurality of snap hooks 19 of which only one is provided with a reference sign. The first housing part 15 comprises a plurality of corresponding receiving sections 20 of which only one is provided with a reference sign. To connect the second housing part 16 to the first housing part 15, the snap hooks 19 engage with the receiving sections 20. The second housing part 16 further has a plurality of guidance elements 21 of which only one is provided with a reference sign. The guidance elements 21 also engage with the receiving sections 20.

Within the first housing part 15 there are provided guidance tracks 22 to 24 (Fig. 8). The number of guidance tracks 22 to 24 is arbitrary. Preferably, there are provided three guidance tracks 22 to 24. The guidance track 22 and the guidance tracks 23, 24 are provided on opposing walls 25, 26 of the first housing part 15. Each guidance track 22 to 24 has a damping element 27 to 29. The damping elements 27 to 29 are made of a flexible material. For example, the damping elements 27 to 29 are made of polyurethane or the like. The housing 14 encloses an interior 30. The guidance tracks 22 to 24 are arranged within this interior 30.

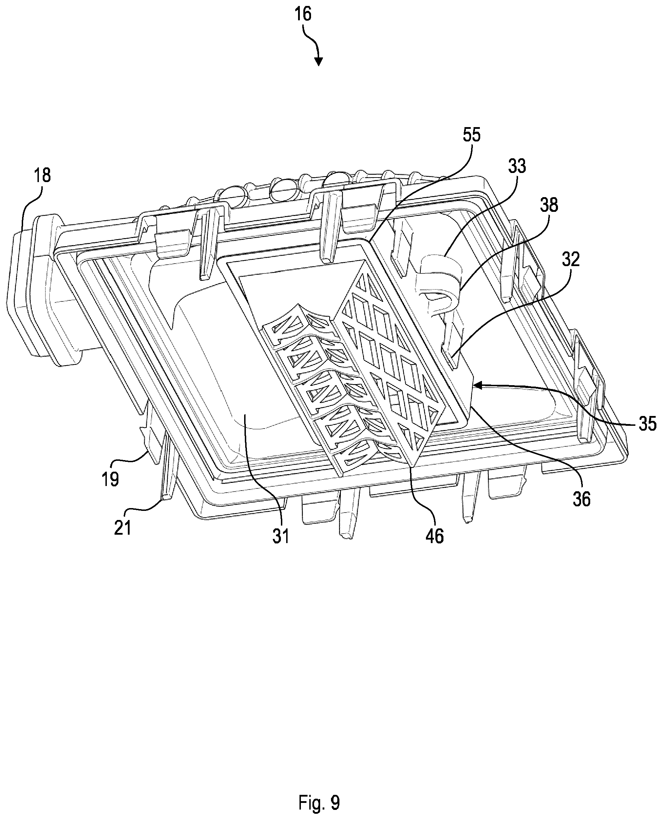

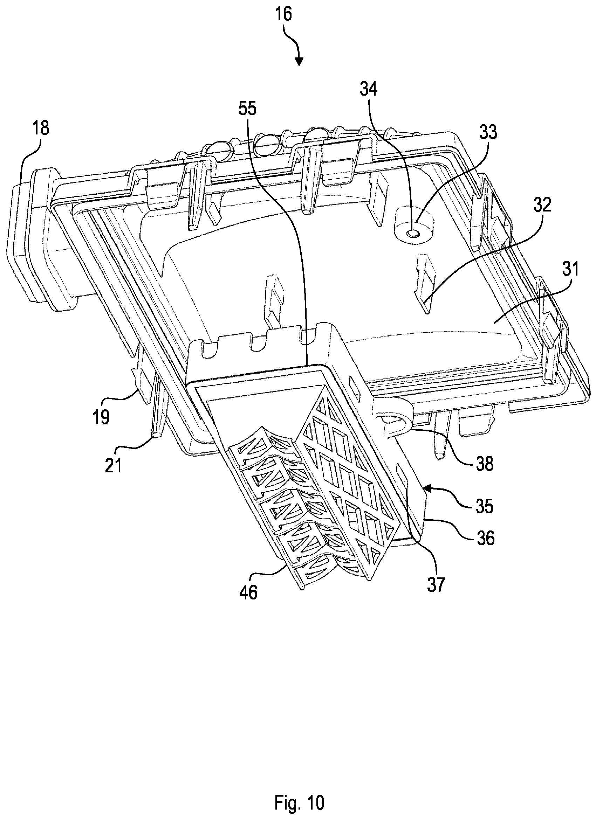

Fig. 9 shows a perspective view of one embodiment of the second housing part 16. Fig. 10 shows an exploded perspective view of the second housing part 16. In the following, Figs. 9 and 10 will be referred to at the same time.

The second housing part 16 has an inner surface 31 that faces the interior 30. From the inner surface 31, a plurality of snap hooks 32, of which only one is provided with a reference sign, protrude into the interior 30. Also a cylindric attachment section 33 protrudes from the inner surface 31 into the interior 30. The attachment section 33 has a central bore 34. The bore 34 can have a thread.

An air heating and guidance mechanism 35 is attached to the second housing part 16. The air heating and guidance mechanism 35 lies against the inner surface 31. The air heating and guidance mechanism 35 is attached to the second housing part 16 by means of the snap hooks 32 and the attachment section 33.

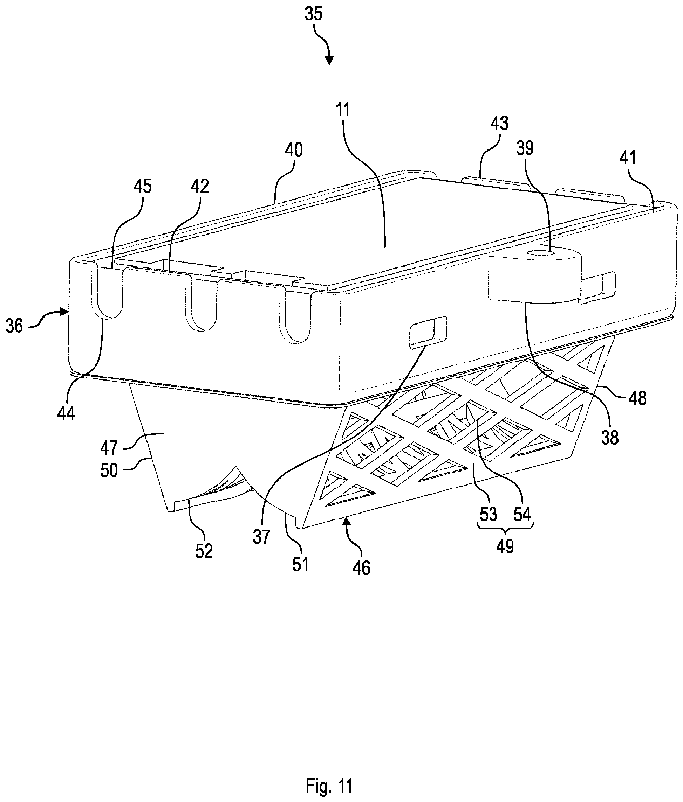

A perspective view of the air heating and guidance mechanism 35 is shown in Fig. 11. The air heating and guidance mechanism 35 comprises a frame 36. The frame 36 has engagement openings 37 that engage with the snap hooks 32 to attach the air heating and guidance mechanism 35 to the second housing part 16. The frame 36 has an attachment section 38. The attachment section 38 has a bore 39. The attachment section 38 of the frame 36 can be bolted to the attachment section 33 of the second housing part 16.

The frame 36 is rectangular and comprises two opposing side walls 40, 41 and two opposing face walls 42, 43. The engagement openings 37 are provided at the side walls 40, 41. The attachment section 38 is provided at the side wall 41. The face walls 42, 43 have openings 44 of which only one is provided with a reference sign.

The frame 36 receives a first heating element 11 as mentioned before. However, in this case, the first heating element 11 is not a heating wire but a plate-shaped heating element. In particular, the first heating element 11 is a positive temperature coefficient (PTC) heater. The first heating element 11 is glued into the frame 36 by means of an adhesive 45. The adhesive 45 is temperature resistant.

The air heating and guidance mechanism 35 further comprises an air guidance element 46. The air guidance element 46 and the frame 36 can be formed integrally. The air guidance element 46 comprises two opposing face walls 47, 48. Furthermore, the air guidance element 46 comprises two opposing first side walls 49, 50 and two opposing second side walls 51, 52. The first side walls 49, 50 are inclined to each other. The second side walls 51, 52 are also inclined to each other. The side walls 49 to 52 form a W-shaped cross section of the air guidance element 46. The side walls 49 to 52 are truss-shaped and comprise a plurality of struts 53 and openings 54 that are separated from each other by means of the struts 53.

A flexible sealing element 55 (Fig. 9 and 10) runs around the air guidance element 46. The sealing element 55 is rectangular.

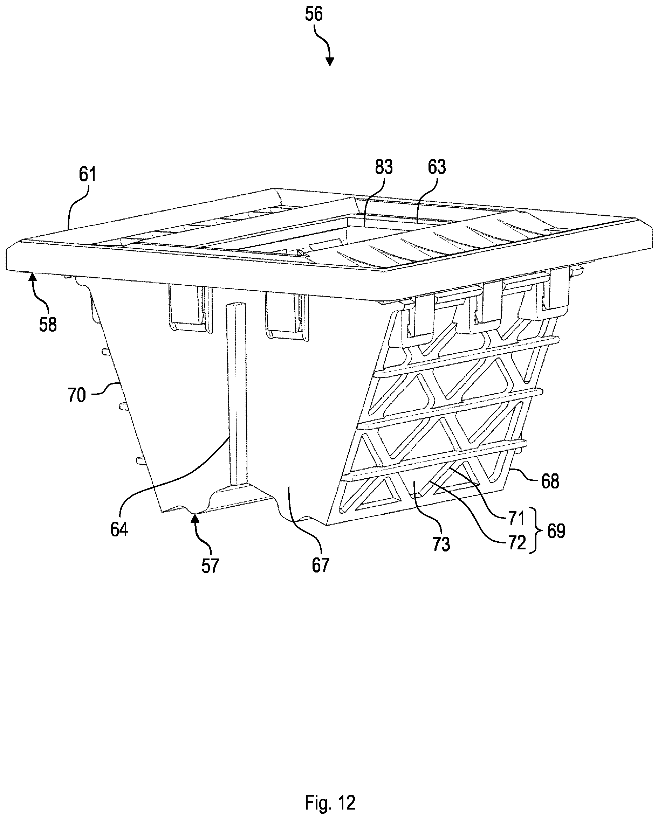

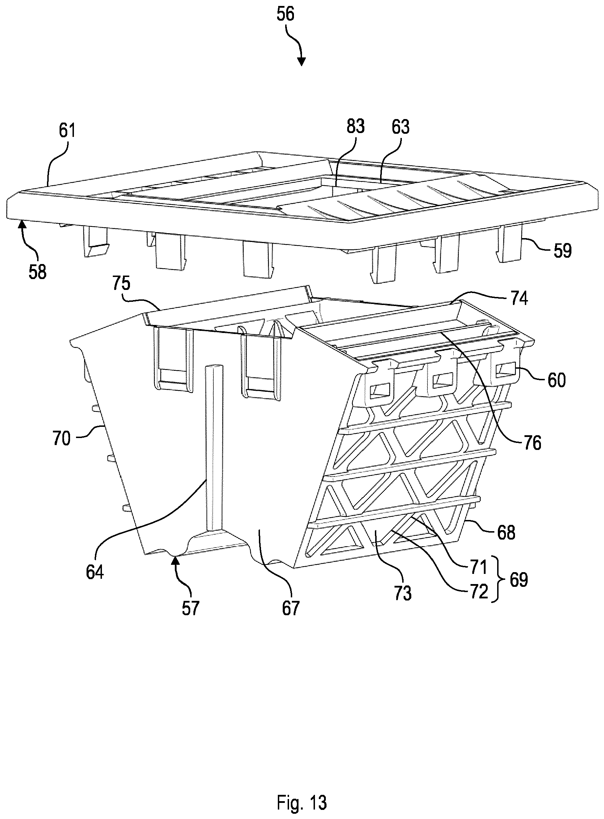

Now returning to Figs. Figs. 3 to 8, the first sorption filter device 6 has a sorption filter element 56. The sorption filter element 56 is exchangeable. The sorption filter element 56 comprises the sorbents 8, 9. A schematic persepective view of the sorption filter element 56 is shown in Fig. 12. Fig. 13 shows a schematic exploded view of the sorption filter element 56.

The directions x, y, z can be assigned to the sorption filter element 56. The sorption filter element 56 comprises a body 57 and a cover 58. The cover 58 comprises a plurality of snap hooks 59 of which only one is provided with a reference sign. The snap hooks 59 engage with receiving sections 60 of the body 57. Only one of the receiving sections 60 is provided with a reference sign. The cover 58 comprises a sealing element 61 that lies against a sealing surface 62 (Fig. 6) of the second housing part 16. The sealing element 61 is flexible. The cover 58 has a sealing surface 63. The sealing element 55 lies against the sealing surface 63.

The sealing element 61 and the the sealing surface 62 form a first sealing plane E1 (Fig. 6). The sealing element 55 and the sealing surface 63 form a second sealing plane E2. When seen along the height direction y, the sealing planes E1, E2 are spaced apart from each other in a distance d. The sealing planes E1, E2 together form a sealing interface F of the sorption filter element 56.

The body 57 comprises guidance tracks 64 to 66 (Fig. 8) that engage with the guidance tracks 22 to 24 of the first housing part 15. The body 57 has two face walls 67, 68. The guidance track 64 protrudes from the face wall 67. The guidance tracks 65, 66 protrude from the face wall 68. Two outer side walls 69, 70 are inclined to each other. The outer side walls 69, 70 are truss-shaped. The outer side walls 69, 70 comprises struts 71 and openings 72. The openings 72 are separated from each other by means of the struts 71. The openings 72 are covered by means of a fluid permeable material 73. The material 73 can be a fiber material, for example a fleece material.

The body 57 further has inner side walls 74, 75. The inner side walls 74, 75 are inclined to each other. The side walls 69, 74 are parallel to each other. The side walls 70, 75 are parallel to each other. The inner side walls 74, 75 are, as the outer side walls 69, 70, fluid permeable. This means that also the inner side walls 74, 75 are truss-shaped and comprise struts 71 and openings 72 as mentioned before. The inner side walls 74, 75 are covered with the material 73 too. The sorbents 8, 9 are placed between the side walls 69, 74 and the side walls 70, 75. The sorbents 8, 9 are part of a first sorption body B1 and a second sorption body B2. The material 73 is part of the sorption bodies B1, B2.

The sorbents 8, 9 can be mixed as shown in Fig. 2 or, as shown in Fig. 8, be separated from each other by means of a separating material 76, 77. There are provided guidance tracks 78 to 81 for receiving the separating material 76, 77. The separating material 76, 77 is fluid permeable. The inner side walls 74, 75 enclose an intermediate space 82 (Fig. 6) that receives the air guidance element 46. The cover 58 comprises an opening 83 (Figs. 12 and 13). The air guidance element 46 is guided through that opening 83 into the intermediate space 82. The side walls 49, 50 of the air guidance element 46 enclose an inner space 84 (Fig. 6) that is separated from the intermediate space 82.

In the sorption mode M1, raw air RO loaded with CO2 and H2O from the passenger compartment 3 is fed to the first sorption filter device 6 by means of the first air inlet/outlet 17. The raw air RO is passed through the first sorption filter device 6. The purified raw air RO is fed back to the passenger compartment 3 as clean air RL via the second air inlet/outlet 18.

In the desorption mode M2, unloaded regeneration air R1 is supplied to the first sorption filter device 6 via the second air inlet/oulet 18. The unloaded regeneration air R1 passes the air heating and guidance mechanism 35 which applies heat Q to the unloaded regeneration air R1. The unloaded regeneration air R1 passes the sorption filter element 56 and leaves the first sorption filter device 6 via the first air inlet/outlet 17 as as loaded regeneration air R2.

### List of Reference Numbers

- 1: Motor vehicle
- 2: Body
- 3: Volume/passenger compartment
- 4: HVAC system
- 5: Mechanism
- 6: Sorption filter device
- 7: Sorption filter device
- 8: Sorbent
- 9: Sorbent
- 10: Blower
- 11: Heating element
- 12: Blower
- 13: Heating element
- 14: Housing
- 15: First housing part
- 16: Second housing part
- 17: Air inlet/outlet
- 18: Air inlet/outlet
- 19: Snap hook
- 20: Receiving section
- 21: Guidance element
- 22: Guidance track
- 23: Guidance track
- 24: Guidance track
- 25: Wall
- 26: Wall
- 27: Damping element
- 28: Damping element
- 29: Damping element
- 30: Interior
- 31: Surface
- 32: Snap hook
- 33: Attachment section
- 34: Bore
- 35: Air heating and guidance mechanism
- 36: Frame
- 37: Engagement opening
- 38: Attachment section
- 39: Bore
- 40: Side wall
- 41: Side wall
- 42: Face wall
- 43: Face wall
- 44: Opening
- 45: Adhesive
- 46: Air guidance element
- 47: Face wall
- 48: Face wall
- 49: Side wall
- 50: Side wall
- 51: Side wall
- 52: Side wall
- 53: Strut
- 54: Opening
- 55: Sealing element
- 56: Sorption filter element
- 57: Body
- 58: Cover
- 59: Snap hook
- 60: Receiving section
- 61: Sealing element
- 62: Sealing surface
- 63: Sealing surface
- 64: Guidance track
- 65: Guidance track
- 66: Guidance track
- 67: Face wall
- 68: Face wall
- 69: Side wall
- 70: Side wall
- 71: Strut
- 72: Opening
- 73: Material
- 74: Side wall
- 75: Side wall
- 76: Separating material
- 77: Separating material
- 78: Guidance track
- 79: Guidance track
- 80: Guidance track
- 81: Guidance track
- 82: Space
- 83: Opening
- 84: Space
- B1: Sorption body
- B2: Sorption body
- d: Distance
- F: Interface
- M1: Sorption mode
- M2: Desorption mode
- Q: Heat
- R1: Regeneration air
- R2: Regeneration air
- RL: Clean air
- RO: Raw air
- U: Environment
- x: Width direction
- y: Height direction
- z: Depth direction

## Claims

1. Sorption filter element (56) for a sorption filter device (6, 7), comprising at least two sorption bodies (B1, B2) with at least one sorbent (8, 9), wherein the sorption bodies (B1, B2) are arranged in a V-shape, wherein the sorption bodies (B1, B2) enclose an intermediate space (82) that is open at a head side thereof, wherein the sorption filter element (56) comprises an outer sealing element (61) that runs around an outer circumference of the head side, wherein the sorption filter element (56) comprises a circumferential inner sealing surface (63) that is arranged at the head side, wherein the inner sealing surface (63) runs around a circumferene delimiting the intermediate space (82) at the head side, and wherein the inner sealing surface (63) is axially set back relative to the outer sealing element (61), and **characterised in that**,
the inner sealing surface (63) is placed within the intermediate space (82).

2. Sorption filter element according to claim 1, wherein the sorption filter element (56) comprises two spaced face walls (67, 68) that in particular connect the sorption bodies (B1, B2) to each other.

3. Sorption filter element according to claim 2, wherein the inner sealing surface (63) is formed at least partially by a collar section of the face walls (67, 68) being drawn into the intermediate space (82) on an inside thereof.

4. Sorption filter element according to claim 2 or 3, wherein the sorption filter element (56) comprises at least two grid-like side walls (69, 70, 74, 75), wherein each side wall (69, 70, 74, 75) neighbors at least one of the sorption bodies (B1, B2) and/or at least partially encloses the sorbent (8, 9).

5. Sorption filter element according to claim 4, wherein the side walls (69, 70, 74, 75) are covered with an air permeable material (73) that faces the sorbent (8, 9), wherein the material (73) holds back the sorbent (8, 9).

6. Sorption filter element according to claim 4 or 5, wherein the face walls (67, 68) and the side walls (69, 70, 74, 75) are formed integrally.

7. Sorption filter element according to one of claims 4 to 6, wherein the sorption filter element (56) comprises a base body comprising at least the face walls (67, 68) and the side walls (69, 70, 74, 75), wherein the sorption bodies (B1, B2) are replaceably held at the base body, in particular between the face walls (67, 68) and neighboring at least one of the side walls (69, 70, 74, 75).

8. Sorption filter element according to one of claims 2 to 7, wherein at least one of the face walls (67, 68) comprises a guidance track (64 - 66), wherein the guidance track (64 - 66) is configured to engage with a housing-sided guidance track (78 - 81) for axially guiding the sorption filter element (56).

9. Sorption filter element according to one of claims 1 to 8, wherein the outer sealing element (61) and/or the inner sealing surface (63) is rectangular.

10. Sorption filter element according to one of claims 1 to 9, wherein the sorbent (8, 9) is bulk material.

11. Sorption filter element according to one of claims 1 to 10, wherein the sorption filter element (56) comprises a first sorbent (8) and a second sorbent (9).

12. Sorption filter element according to claim 11, wherein the first sorbent (8) is mixed with the second sorbent (9).

13. Sorption filter element according to claim 11, wherein the first sorbent (8) and the second sorbent (9) are provided in layers being separated from each other by means of an air permeable separating material (76, 77).

14. Sorption filter device (6, 7), comprising at least one sorption filter element (56) according to one of claims 1 to 13, and a housing (14), wherein the housing (14) comprises a first housing part (15) for receiving the sorption filter element (56) and a second housing part (16), wherein the second housing part (16) is detachably connectable to the first housing part (15), wherein the second housing part (16) closes the housing (14) in a closed state thereof, wherein the sorption filter element (56) is received in the first housing part (15) in such a way that the head side faces the second housing part (16), wherein the housing (14) comprises at least one first air inlet/outlet (17) and at least one second air inlet/outlet (18), wherein the sorption filter element (56) is arranged between the first air inlet/outlet (17) and the second air inlet/outlet (18) in a fluidic way, wherein at least one of the first air inlet/outlet (17) and the second air inlet/outlet (18) is provided at the second housing part (16), wherein the sorption filter device (6, 7) comprises an air guidance element (46) that is arranged between the sorption filter element (56) and the second housing part (16), and wherein the air guidance element (46) protrudes into the intermediate space (82) at the head side in a mounted state of the second housing part (16).

15. Use of a sorption filter element (56) according to one of claims 1 to 13 in a sorption filter device (6, 7) according to claim 14.

16. Use of a sorption filter device (6, 7) according to claim 14 in a system for reducing the CO2-content within a passenger compartment (3).

## Patentansprüche

1. Sorptionsfilterelement (56) für eine Sorptionsfiltervorrichtung (6, 7), umfassend mindestens zwei Sorptionskörper (B1, B2) mit mindestens einem Sorptionsmittel (8, 9), wobei die Sorptionskörper (B1, B2) V-förmig angeordnet sind, wobei die Sorptionskörper (B1, B2) einen Zwischenraum (82) umschließen, der an einer Kopfseite davon offen ist, wobei das Sorptionsfilterelement (56) ein Außendichtungselement (61) umfasst, das um einen Außenumfang der Kopfseite herum verläuft, wobei das Sorptionsfilterelement (56) eine umlaufende Innendichtfläche (63) umfasst, die an der Kopfseite angeordnet ist, wobei die Innendichtfläche (63) um einen Umfang verläuft, der den Zwischenraum (82) an der Kopfseite begrenzt, und wobei die Innendichtfläche (63) gegenüber dem Außendichtungselement (61) axial zurückgesetzt ist, und **dadurch gekennzeichnet, dass** die innere Dichtfläche (63) innerhalb des Zwischenraums (82) angeordnet ist.

2. Sorptionsfilterelement nach Anspruch 1, wobei das Sorptionsfilterelement (56) zwei beabstandete Stirnwände (67, 68) umfasst, die insbesondere die Sorptionskörper (B1, B2) miteinander verbinden.

3. Sorptionsfilterelement nach Anspruch 2, wobei die Innendichtfläche (63) zumindest teilweise dadurch gebildet ist, dass ein Kragenabschnitt der Stirnwände (67, 68) in den Zwischenraum (82) auf dessen Innenseite gezogen ist.

4. Sorptionsfilterelement nach Anspruch 2 oder 3, wobei das Sorptionsfilterelement (56) mindestens zwei gitterartige Seitenwände (69, 70, 74, 75) umfasst, wobei jede Seitenwand (69, 70, 74, 75) an mindestens einen der Sorptionskörper (B1, B2) angrenzt und/oder das Sorptionsmittel (8, 9) zumindest teilweise umschließt.

5. Sorptionsfilterelement nach Anspruch 4, wobei die Seitenwände (69, 70, 74, 75) mit einem luftdurchlässigen Material (73) bedeckt sind, das dem Sorptionsmittel (8, 9) zugewandt ist, wobei das Material (73) das Sorptionsmittel (8, 9) zurückhält.

6. Sorptionsfilterelement nach Anspruch 4 oder 5, wobei die Stirnwände (67, 68) und die Seitenwände (69, 70, 74, 75) einstückig ausgebildet sind.

7. Sorptionsfilterelement nach einem der Ansprüche 4 bis 6, wobei das Sorptionsfilterelement (56) einen Grundkörper umfasst, der mindestens die Stirnwände (67, 68) und die Seitenwände (69, 70, 74, 75) umfasst, wobei die Sorptionskörper (B1, B2) austauschbar am Grundkörper gehalten sind, insbesondere zwischen den Stirnwänden (67, 68) und benachbart zu mindestens einer der Seitenwände (69, 70, 74, 75).

8. Sorptionsfilterelement nach einem der Ansprüche 2 bis 7, wobei mindestens eine der Stirnwände (67, 68) eine Führungsspur (64 - 66) umfasst, wobei die Führungsspur (64 - 66) so ausgebildet ist, dass sie mit einer gehäuseseitigen Führungsspur (78 - 81) zur axialen Führung des Sorptionsfilterelements (56) in Eingriff kommt.

9. Sorptionsfilterelement nach einem der Ansprüche 1 bis 8, wobei das Außendichtungselement (61) und/oder die Innendichtfläche (63) rechteckförmig ist.

10. Sorptionsfilterelement nach einem der Ansprüche 1 bis 9, wobei das Sorptionsmittel (8, 9) Schüttgut ist.

11. Sorptionsfiltervorrichtung nach einem der Ansprüche 1 bis 10, wobei das Sorptionsfilterelement (56) ein erstes Sorptionsmittel (8) und ein zweites Sorptionsmittel (9) umfasst.

12. Sorptionsfiltervorrichtung nach Anspruch 11, wobei das erste Sorptionsmittel (8) mit dem zweiten Sorptionsmittel (9) vermischt wird.

13. Sorptionsfilterelement nach Anspruch 11, wobei das erste Sorptionsmittel (8) und das zweite Sorptionsmittel (9) in Schichten vorgesehen sind, die durch ein luftdurchlässiges Trennmaterial (76, 77) voneinander getrennt sind.

14. Sorptionsfiltervorrichtung (6, 7), umfassend mindestens ein Sorptionsfilterelement (56) nach einem der Ansprüche 1 bis 13, und ein Gehäuse (14), wobei das Gehäuse (14) einen ersten Gehäuseteil (15) zur Aufnahme des Sorptionsfilterelements (56) und einen zweiten Gehäuseteil (16) umfasst, wobei das zweite Gehäuseteil (16) lösbar mit dem ersten Gehäuseteil (15) verbindbar ist, wobei das zweite Gehäuseteil (16) das Gehäuse (14) in einem geschlossenen Zustand desselben verschließt, wobei das Sorptionsfilterelement (56) in dem ersten Gehäuseteil (15) derart aufgenommen ist, dass die Kopfseite dem zweiten Gehäuseteil (16) zugewandt ist, wobei das Gehäuse (14) mindestens einen ersten Lufteinlass/Luftauslass (17) und mindestens einen zweiten Lufteinlass/Luftauslass (18) umfasst, wobei das Sorptionsfilterelement (56) zwischen dem ersten Lufteinlass/Luftauslass (17) und dem zweiten Lufteinlass/Luftauslass (18) fluidisch angeordnet ist, wobei mindestens einer von dem ersten Lufteinlass/Luftauslass (17) und dem zweiten Lufteinlass/Luftauslass (18) an dem zweiten Gehäuseteil (16) vorgesehen ist, wobei die Sorptionsfiltervorrichtung (6, 7) ein Luftleitelement (46) umfasst, das zwischen dem Sorptionsfilterelement (56) und dem zweiten Gehäuseteil (16) angeordnet ist, und wobei das Luftleitelement (46) in einem montierten Zustand des zweiten Gehäuseteils (16) in den Zwischenraum (82) an der Kopfseite hineinragt.

15. Verwendung eines Sorptionsfilterelements (56) nach einem der Ansprüche 1 bis 13 in einer Sorptionsfiltervorrichtung (6, 7) nach Anspruch 14.

16. Verwendung einer Sorptionsfiltervorrichtung (6, 7) nach Anspruch 14 in einem System zur Reduzierung des CO2-Gehalts in einem Fahrgastraum (3).

## Revendications

1. Élément de filtre à sorption (56) destiné à un dispositif de filtre à sorption (6, 7), comprenant au moins deux corps de sorption (B1, B2) avec au moins un sorbant (8, 9), dans lequel les corps de sorption (B1, B2) sont agencés sous une forme de V, dans lequel les corps de sorption (B1, B2) renferment un espace intermédiaire (82) qui est ouvert au niveau d'un côté de tête de ceux-ci, dans lequel l'élément de filtre à sorption (56) comprend un élément d'étanchéité externe (61) qui s'étend autour d'une circonférence externe du côté de tête, dans lequel l'élément de filtre à sorption (56) comprend une surface d'étanchéité interne circonférentielle (63) qui est agencée au niveau du côté de tête, dans lequel la surface d'étanchéité interne (63) s'étend autour d'une circonférence délimitant l'espace intermédiaire (82) au niveau du côté de tête, et dans lequel la surface d'étanchéité interne (63) est axialement reculée par rapport à l'élément d'étanchéité externe (61), et **caractérisé en ce que** la surface d'étanchéité interne (63) est placée au sein de l'espace intermédiaire (82).

2. Élément de filtre à sorption selon la revendication 1, dans lequel l'élément de filtre à sorption (56) comprend deux parois frontales (67, 68) espacées qui relient notamment les corps de sorption (B1, B2) les uns aux autres.

3. Élément de filtre à sorption selon la revendication 2, dans lequel la surface d'étanchéité interne (63) est formée au moins partiellement par une section de collier des parois frontales (67, 68) étant tirée dans l'espace intermédiaire (82) sur un intérieur de celui-ci.

4. Élément de filtre à sorption selon la revendication 2 ou 3, dans lequel l'élément de filtre à sorption (56) comprend au moins deux parois latérales (69, 70, 74, 75) de type grille, dans lequel chaque paroi latérale (69, 70, 74, 75) est voisine d'au moins l'un des corps de sorption (B1, B2) et/ou renferme au moins partiellement le sorbant (8, 9).

5. Élément de filtre à sorption selon la revendication 4, dans lequel les parois latérales (69, 70, 74, 75) sont couvertes d'un matériau perméable à l'air (73) qui fait face vers le sorbant (8, 9), dans lequel le matériau (73) retient le sorbant (8, 9).

6. Élément de filtre à sorption selon la revendication 4 ou 5, dans lequel les parois frontales (67, 68) et les parois latérales (69, 70, 74, 75) sont formées d'un seul tenant.

7. Élément de filtre à sorption selon l'une quelconque des revendications 4 à 6, dans lequel l'élément de filtre à sorption (56) comprend un corps de base comprenant au moins les parois frontales (67, 68) et les parois latérales (69, 70, 74, 75), dans lequel les corps de sorption (B1, B2) sont maintenus de manière remplaçable au niveau du corps de base, notamment entre les parois frontales (67, 68) et au voisinage d'au moins l'une des parois latérales (69, 70, 74, 75).

8. Élément de filtre à sorption selon l'une quelconque des revendications 2 à 7, dans lequel au moins l'une des parois frontales (67, 68) comprend une trajectoire de guidage (64 - 66), dans lequel la trajectoire de guidage (64 - 66) est conçue pour venir en prise avec une trajectoire de guidage côté boîtier (78 - 81) permettant de guider axialement l'élément de filtre à sorption (56).

9. Élément de filtre à sorption selon l'une quelconque des revendications 1 à 8, dans lequel l'élément d'étanchéité externe (61) et/ou la surface d'étanchéité interne (63) sont rectangulaires.

10. Élément de filtre à sorption selon l'une quelconque des revendications1 à 9, dans lequel le sorbant (8, 9) est un matériau en vrac.

11. Élément de filtre à sorption selon l'une quelconque des revendications 1 à 10, dans lequel l'élément de filtre à sorption (56) comprend un premier sorbant (8) et un second sorbant (9).

12. Élément de filtre à sorption selon la revendication 11, dans lequel le premier sorbant (8) est mélangé avec le second sorbant (9).

13. Élément de filtre à sorption selon la revendication 11, dans lequel le premier sorbant (8) et le second sorbant (9) sont fournis dans des couches étant séparées les unes des autres au moyen d'un matériau de séparation perméable à l'air (76, 77).

14. Dispositif de filtre à sorption (6, 7), comprenant au moins un élément de filtre à sorption (56) selon l'une quelconque des revendications 1 à 13, et un boîtier (14), dans lequel le boîtier (14) comprend une première partie de boîtier (15) permettant de recevoir l'élément de filtre à sorption (56) et une seconde partie de boîtier (16), dans lequel la seconde partie de boîtier (16) peut être reliée de manière détachable à la première partie de boîtier (15), dans lequel la seconde partie de boîtier (16) ferme le boîtier (14) dans un état fermé de celui-ci, dans lequel l'élément de filtre à sorption (56) est reçu dans la première partie de boîtier (15) d'une manière telle que le côté de tête fait face vers la seconde partie de boîtier (16), dans lequel le boîtier (14) comprend au moins une première entrée/sortie d'air (17) et au moins une seconde entrée/sortie d'air (18), dans lequel l'élément de filtre à sorption (56) est agencé entre la première entrée/sortie d'air (17) et la seconde entrée/sortie d'air (18) d'une manière fluidique, dans lequel au moins l'une parmi la première entrée/sortie d'air (17) et la seconde entrée/sortie d'air (18) est fournie au niveau de la seconde partie de boîtier (16), dans lequel le dispositif de filtre à sorption (6, 7) comprend un élément de guidage d'air (46) qui est agencé entre l'élément de filtre à sorption (56) et la seconde partie de boîtier (16), et dans lequel l'élément de guidage d'air (46) fait saillie dans l'espace intermédiaire (82) au niveau du côté de tête dans un état monté de la seconde partie de boîtier (16).

15. Utilisation d'un élément de filtre à sorption (56) selon l'une quelconque des revendications 1 à 13 dans un dispositif de filtre à sorption (6, 7) selon la revendication 14.

16. Utilisation d'un dispositif de filtre à sorption (6, 7) selon la revendication 14 dans un système permettant de réduire la teneur en CO2 au sein d'un habitacle (3).
